# EUROPEAN PATENT APPLICATION

(11) **EP 4 759 940 A1**
(43) Date of publication of application: **17.06.2026**
(21) Application number: 24863024.6
(22) Date of filing: 31.07.2024
(51) Int. Cl.: C12Q 1/6818

(54) **TECHNOLOGY FOR DETECTING TARGET NUCLEIC ACID SEQUENCE BY USING ARGONAUTE PROTEIN AND ARTIFICIAL NUCLEIC ACID CIRCUIT**

(30) Priority: 08.09.2023 KR 20230119796
(71) Applicant: Korea Research Institute of Bioscience and Biotechnology, Daejeon 34141 (KR)
(72) Inventor: KANG, Tae Joon, Daejeon 34141 (KR); JANG, Hyo Won, Daejeon 34141 (KR); SONG, Ja Yeon, Daejeon 34141 (KR); LIM, Eun Kyung, Daejeon 34141 (KR); JUNG, Ju Yeon, Daejeon 34141 (KR)
(74) Representative: Symbiosis IP Limited
(86) International application number: PCT/KR2024/011204
(87) International publication number: WO 2025/053442

(57) **Abstract**

The present disclosure relates to a technology for detecting a target nucleic acid by using an argonaute protein and an artificial nucleic acid circuit. The system according to the present disclosure can specifically detect target pathogens added to a human sample with 100% clinical sensitivity without nucleic acid amplification and separate extraction steps, and can directly identify target pathogens collected from the surface of an object by using the system together with a three-dimensional nanopillar array structure. In addition, since a trigger sequence cleaved by an Ago protein is designed to be used as a guide, a single protein is used, and thus manufacturing is simpler than that of a conventional nucleic acid detection circuit, and cost are reduced in that target pathogens can be detected with high sensitivity even with trace amounts of target nucleic acid sequences as long as the Ago protein, a reporter, and a reporter complement according to the system of the present disclosure are not depleted. In addition, the system according to the present disclosure proceeds in a single step, and can be easily applied to the detection of various target nucleic acid sequences by appropriately changing a nucleic acid recognition site to match a target pathogen.

## Description

### [Technical Field]

The present disclosure relates to a target nucleic acid sequence detection technology using an Argonaute protein and an artificial nucleic acid circuit.

### [Background Art]

Infection of Carbapenemase-producing Enterobacteriaceae (CPE) has emerged as a significant public health issue worldwide due to rapid transmission, high mortality rates, and limited treatment options. CPE is a group of Gram-negative bacteria including KPC (Klebsiella pneumoniae carbapenemase), IMP (Imipenemase), NDM (New-Delhi metallo-beta-lactamase), VIM (Verona Integron-encoded Metallo-beta-lactamase), and OXA-48 (Oxacillinase-48), which exhibit hydrolytic activity against carbapenem antibiotics used as a last resort for multidrug-resistant strain infections. Currently, as the prevalence of CPE infection increases in medical environments and local communities, there is an urgent need for rapid, accurate, and cost-effective diagnostic methods to identify these resistant variants.

Existing CPE detection methods, such as culture-based detection methods and polymerase chain reaction (PCR), reveal limitations in terms of long processing times, labor intensiveness, and the need for special equipment and skilled personnel. Accordingly, isothermal nucleic acid amplification methods are attracting attention as alternative diagnostic techniques that overcome these problems and enable appropriate treatment by detecting CPE infections in a timely manner. However, since the composition of an isothermal amplification mixture is relatively very complex, it is often incompatible with clinical samples. To overcome this, most methods use additional purification processes, equipment, and reagents; however, the resulting increase in cost and complexity ultimately limits the practical applicability of isothermal nucleic acid diagnosis.

Recently, endonucleases have been attracting attention as useful tools for identifying target nucleic acids due to their sequence-specific catalytic activity. In particular, the CRISPR/Cas system is receiving great attention for its potential in molecular diagnostics; among them, CRISPR/Cas12 and CRISPR/Cas13 systems have demonstrated excellent potential for diagnosis due to their collateral cleavage activity. Upon recognizing and binding to a target sequence, Cas12 and Cas13 have activities of non-specifically cleaving nearby single-stranded DNA and RNA molecules, respectively, and such collateral cleavage activity can be utilized for signal amplification, thereby detecting target nucleic acids with high sensitivity. Despite these possibilities, the diagnostic application of the CRISPR/Cas system faces several problems, such as increased cost and instability due to the use of guide RNA, and the requirement that the cleavage site preferred by the Cas protein must be adjacent to the target sequence. In order to solve these limitations, strategies such as the development of alternative endonucleases and the utilization of chemically modified guide RNA are being actively researched.

Accordingly, the present inventors have completed the present disclosure by developing a technology that solves the above problems by using an Argonaute protein and an artificial nucleic acid circuit, while simultaneously exhibiting an excellent target nucleic acid sequence detection effect.

### [Disclosure]

### [Technical Problem]

One object of the present disclosure is to provide a composition for detecting a target nucleic acid comprising: an Argonaute protein; a first guide nucleic acid (G1); a second guide nucleic acid (G2); a reporter sequence to which a quencher and a fluorophore are coupled; and a reporter complement.

Another object of the present disclosure is to provide a kit comprising the composition for detecting a target nucleic acid described above and a method for detecting a target nucleic acid sequence using the same.

### [Technical Solution]

This will be described in detail as follows. Meanwhile, each description and embodiment disclosed in the present disclosure may also be applied to other descriptions and embodiments. That is, all combinations of various elements disclosed in the present disclosure fall within the scope of the present disclosure. In addition, it cannot be considered that the scope of the present disclosure is limited by the specific descriptions described below.

In addition, terms used in this specification are used only for the purpose of description and should not be construed as intended to be limiting. Singular expressions include plural expressions unless the context clearly indicates otherwise. In this specification, terms such as "comprise" or "have" are intended to designate that features, numbers, steps, operations, components, parts, or combinations thereof described in the specification exist, but should be understood as not precluding the possibility of the existence or addition of one or more other features, numbers, steps, operations, components, parts, or combinations thereof.

Also, unless defined otherwise, all terms used herein, including technical or scientific terms, have the same meaning as commonly understood by one of ordinary skill in the art to which the embodiment belongs. Terms such as those defined in commonly used dictionaries should be interpreted as having meanings consistent with the meanings in the context of the related art, and are not interpreted in an ideal or excessively formal sense unless explicitly defined in the present application.

In addition, in the following description, the description of overlapping content has been omitted to prevent congestion of overlapping content. That is, the content of the invention is not limited only to the following content, and the content of the invention should be interpreted according to the overall content of the invention.

Endonucleases are attracting attention as useful tools for identifying target nucleic acids due to their sequence-specific catalytic activity. In particular, the CRISPR/Cas system is receiving great attention for its potential in molecular diagnostics; among them, CRISPR/Cas12 and CRISPR/Cas13 systems have demonstrated excellent potential for diagnosis due to their collateral cleavage activity. However, despite these possibilities, the diagnostic application of the CRISPR/Cas system faces several problems, such as increased cost and instability due to the use of guide RNA, and the requirement that the cleavage site preferred by the Cas protein must be adjacent to the target sequence.

Meanwhile, Argonaute proteins, named after their homology with eukaryotic Argonaute family proteins, exhibit unique characteristics applicable to molecular diagnostics. One such characteristic is the ability to cleave a target nucleic acid without a special sequence such as a protospacer adjacent motif (PAM) used by Cas proteins for cleavage of a target nucleic acid. Instead, the Argonaute protein precisely cleaves the target nucleic acid by forming base pairs with a short nucleic acid strand between the 10th and 11th positions of the guide DNA, making it easy to design relatively cost-effective and versatile molecular diagnostic techniques.

More specifically, the "Argonaute protein" may be derived from Argonaute present in various organisms such as prokaryotes, eukaryotes, and archaea, and may be derived from Argonaute present in various living organisms without limitation, ranging from bacteria to animals, plants, and humans. Preferably, a protein derived from a thermophilic microorganism is preferred. That is, the thermophilic Argonaute protein is a protein derived from a thermophilic microorganism and shows high stability in a high-temperature environment, thereby improving the robustness of the Argonaute protein-based nucleic acid detection method.

For example, the Argonaute protein may be derived from one or more selected from the group consisting of hAgo2 (from Human) Q9UKV8, TtAgo (from Thermus thermophilus) Q746M7, PfAgo (from Pyrococcus furiosus) Q8U3D2, and CbAgo (from Clostridium butyricum). Preferably, the thermophilic Argonaute protein may be TtAgo (from Thermus thermophilus).

The present disclosure intends to present a new DNA detection technology that combines an artificial nucleic acid circuit and the highly systematic sequence-guided cleavage activity of an Argonaute protein.

Accordingly, the present disclosure provides a composition for detecting a target nucleic acid comprising: an Argonaute protein (Ago); a first guide nucleic acid (G1); a second guide nucleic acid (G2); a reporter sequence to which a quencher and a fluorophore are coupled; and a reporter complement, wherein the reporter complement consists of the same sequence as the reporter sequence to which the quencher and the fluorophore are not coupled, and wherein a phosphate is attached to the 5' end of the first guide nucleic acid, the second guide nucleic acid, or both.

In this case, since the Argonaute protein cannot utilize the first guide nucleic acid or the second guide nucleic acid as a guide nucleic acid if a phosphate is not attached to the first guide nucleic acid or the second guide nucleic acid, or both, the attachment of a phosphate to the 5' end of the first guide nucleic acid and/or the second guide nucleic acid is an essential component.

At this time, it is preferable that the first guide nucleic acid and/or the second guide nucleic acid further have one or more of the following conditions in the design thereof:
1) Designed so that Trigger 1 (T1) and Trigger 2 (T2), which are reaction products cleaved by the Argonaute protein, can act as separate guide nucleic acids,
2) Consist of 16 to 18 bases,
3) Have a CG content between 10% and 30%,
4) Thymine be located at the first base sequence of the nucleic acid, and
5) The 12th base sequence of the nucleic acid not be adenine.

As described above, in order for the first guide nucleic acid and/or the second guide nucleic acid to be utilized as a guide nucleic acid, the condition as a guide nucleic acid is satisfied if a phosphate is essentially attached to the 5' end.

In this case, since the Argonaute protein recognizes the first guide nucleic acid and/or the second guide nucleic acid as a guide, and Trigger 1 and Trigger 2 generated by cleavage thereby naturally take a form in which a phosphate is attached to the 5' end, the Trigger 1 and Trigger 2 described above can function as separate guide nucleic acids.

However, in addition to the condition of attaching a phosphate to the 5' end of the first guide nucleic acid and/or the second guide nucleic acid, since the performance of the guide nucleic acid improves as more of the conditions of 1) to 5) are satisfied, it is preferable that the first guide nucleic acid and/or the second guide nucleic acid satisfy one or more of the above conditions in the design thereof.

In this case, the quencher binding to the reporter sequence may be any one selected from the group consisting of DABCYL, BHQ, ECLIPSE, and TAMRA, and the fluorophore may be any one selected from the group consisting of ALEX-350, FAM, VIC, TET, CAL Fluor^{®} Gold 540, JOE, HEX, CAL Fluor Orange 560, TAMRA, CAL Fluor Red 590, ROX, CAL Fluor Red 610, TEXAS RED, CAL Fluor Red 635, Quasar 670, CY3, CY5, CY5.5, and Quasar 705.

According to one embodiment of the present disclosure, since binding the quencher and the fluorophore only to the reporter nucleic acid exhibited significantly higher detection specificity and sensitivity when applying the Argonaute protein and artificial nucleic acid circuit (ANCA) method compared to binding them to both the reporter nucleic acid and the reporter complement or only to the reporter complement, the quencher and the fluorophore are preferably coupled only to the reporter nucleic acid in the present disclosure.

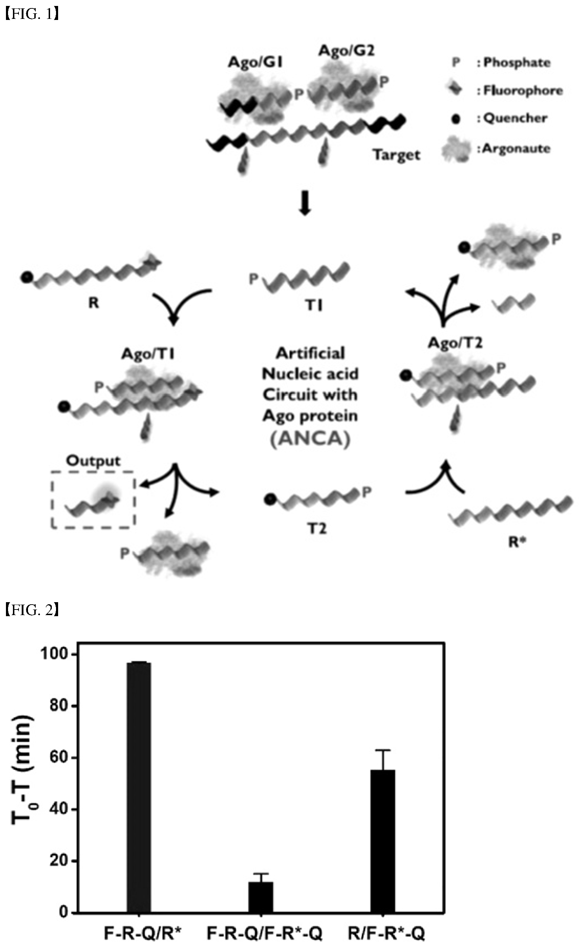

The reaction progress sequence of the Argonaute protein and the artificial nucleic acid circuit (ANCA) using the composition according to the present disclosure is briefly introduced as follows:
1) The ANCA reaction begins as the Argonaute protein (Ago) forms a complex with each of the first guide nucleic acid (G1) and the second guide nucleic acid (G2) and recognizes the target nucleic acid. That is, the Ago/G1 and Ago/G2 complexes hybridize with the complementary target DNA sequence and then cleave the target nucleic acid between the 10th and 11th bases from the 5' end of each guide nucleic acid (indicated by red symbols in FIG. 1). As a result, a first trigger (T1), which is a short nucleic acid fragment, is generated from the target DNA.
2) Since the first trigger (T1) can itself be utilized as another guide nucleic acid, an Ago/T1 complex is formed accordingly; this complex recognizes and cleaves the reporter nucleic acid (R), releasing reaction products (Output), which are two short DNA fragments, and a second trigger (T2).
3) As the reporter nucleic acid is cleaved by the Argonaute protein, the reaction product (Output) generates a fluorescence signal (indicated by the dotted box in FIG. 1), and the second trigger (T2) binds again to the Argonaute protein to form an Ago/T2 complex.
4) The newly formed Ago/T2 complex recognizes and cleaves the reporter complement (R*) to generate the first trigger (T1), thereby completing a repetitively circulating positive feedback loop. That is, through this positive feedback loop, the cleavage reaction is repeatedly performed in the presence of the target nucleic acid, and as the reaction proceeds, the reaction product (Output) is generated at a rapid rate, causing high specificity and an exponential signal amplification reaction. Finally, by monitoring the change in the fluorescence signal, the presence or absence of the target nucleic acid in the sample can be confirmed.

In this case, since the ANCA method uses a single protein, manufacturing is simple and costs are reduced compared to existing nucleic acid detection methods. In addition, this reaction proceeds in a single step, and if the target nucleic acid is known, it can be easily applied to the detection of various target nucleic acids (substances) by changing the nucleic acid recognition site.

In the present disclosure, as one embodiment, the applicability of the ANCA method according to the present disclosure is confirmed by applying the ANCA method only to antibiotic-resistant strains (CPE) producing KPC or IMP; however, since the ANCA method according to the present disclosure can be easily applied without limitation to the type of nucleic acid, strain, or virus as long as its sequence is known, there is no separate limitation on the type of target nucleic acid.

In particular, according to one specific embodiment of the present disclosure, antibiotic-resistant strains (CPE) producing KPC or IMP were successfully detected by using the composition of the present disclosure, and antibiotic-resistant strains (CPE) added to human samples such as urine and blood could be detected without nucleic acid amplification and extraction steps. In addition, the ANCA method was applied to diagnose patients infected with antibiotic-resistant strains (CPE) using rectal swab samples, recording clinical sensitivity and specificity of 100%, respectively.

Therefore, the ANCA method has significant potential for on-site diagnosis of strains, and by facilitating patient treatment in the early stages of strain infection, it can ultimately increase diagnostic accuracy in clinical environments and improve treatment outcomes.

For example, such detection of a target nucleic acid may preferably be for detecting a nucleic acid regarding a pathogen. The term "pathogen" refers to an organism capable of causing disease by directly damaging the host's tissues or producing toxins, and examples thereof include bacteria, viruses, fungi, and other parasites.

The target nucleic acid detection technology according to the present disclosure detects nucleic acids present in a pathogen with high sensitivity and accuracy, thereby providing specific information on whether the target pathogen is infected or whether the pathogen is present.

Accordingly, the present disclosure also provides a composition for detecting a pathogen comprising: an Argonaute protein; a first guide nucleic acid (G1); a second guide nucleic acid (G2); a reporter sequence to which a quencher and a fluorophore are coupled; and a reporter complement, a kit, and a method for detecting a pathogen using the same. Exemplary bacterial genera of pathogens are Staphylococcus, Streptococcus, Pseudomonas, Escherichia, Salmonella, Helicobacter, Neisseria, Campylobacter, Chlamydia, Clostridium, Vibrio, Treponema, Escherichia coli, Mycobacterium, Klebsiella, Actinomyces, Bacterioides, Bordetella, Borrelia, Brucella, Corynebacterium, Diplococcus, Enterobacter, Fusobacterium, Leptospira, Listeria, Pasteurella, Proteus, Rickettsia, Shigella, Sphaerophorus, Acinetobacter, Aeromonas Burkholderia, Campylobacter, Corynebacterium, Enterococcus, Erwinia, Francisella, Haemophilus, Helicobacter, Legionella, Leptospira, Listeria, Mycoplasma, Neisseria, Veillonella, Vibrio, Coxiella, IMP (Imipenemase), New-Delhi metallo-beta-lactamase (NDM), VIM (Verona Integron-encoded Metallo-beta-lactamase), OXA-48 (Oxacillinase-48), or Yersinia.

Meanwhile, the present disclosure provides a kit for detecting a target nucleic acid comprising the composition described above. Specifically, the kit according to the present disclosure comprises a composition for detecting a target nucleic acid comprising: an Argonaute protein (Ago); a first guide nucleic acid (G1); a second guide nucleic acid (G2); a reporter sequence to which a quencher and a fluorophore are coupled; and a reporter complement, wherein the reporter complement consists of the same sequence as the reporter sequence to which the quencher and the fluorophore are not coupled, and wherein a phosphate is attached to the 5' end of the first guide nucleic acid, the second guide nucleic acid, or both.

In one embodiment of the present disclosure, optimal conditions for the ANCA reaction were established. Therefore, the kit according to the present disclosure preferably comprises 150 to 400 nM of the Argonaute protein; 250 to 1,000 nM of the reporter sequence; and 250 to 1,000 nM of the reporter complement; and most preferably comprises 200 nM of the Argonaute protein; 500 nM of the reporter sequence; and 500 nM of the reporter complement.

In addition, the kit preferably further comprises a reaction buffer containing MgCl₂, wherein the MgCl₂ preferably has a concentration of 5 to 30 mM, and most preferably has a concentration of 10 mM.

In addition, the kit may further comprise a collection tool for non-invasively collecting a sample. Such a collection tool may be, for example, at least one selected from the group consisting of an absorbent pad, a swab, a pipette, a spoon, a swap, and a toothpick.

Preferably, it comprises a swab. More specifically, it may further comprise a 3D nanopillar array swab. The 3D nanopillar array swab is a swab specially developed for direct application to a 3D nanopillar array. The 3D nanopillar array swab can be manufactured through a method of replicating a nanopillar array and growing polyaniline nanofibers thereon; this swab can effectively collect strains in a quantitative range of 10² to 10⁷ CFU/ml, and it was confirmed that the maximum capture range reaches 75 strains/100 µm². Such high collection efficiency and wide collection range can improve the sensitivity and accuracy of strain detection.

According to one embodiment of the present disclosure, the combination of the ANCA method and the 3D nanopillar array swab according to the present disclosure was able to improve the detection efficiency of antibiotic-resistant strains (CPE). The unique characteristics of the 3D nanostructure irreversibly collect harmful strains, ensuring a safe diagnostic procedure. Therefore, the integrated use of the ANCA method and the 3D nanopillar array swab can lead to promising developments in diagnostic tools for various pathogens (including strains and viruses, etc.) including antibiotic-resistant strains.

According to one embodiment of the present disclosure, antibiotic-resistant CPE could be directly detected from a rectal swab (3D nanopillar array swab) collected by a patient for the first time through the ANCA method, and these results are very impressive. In addition, in one embodiment of the present disclosure, it was demonstrated that diagnostic results using a rectal swab were more accurate than results obtained from a transport medium in which the swab was immersed. That is, since the possibility of sample contamination can be avoided and the spread of strains can be suppressed by directly applying the clinical sample obtained using the 3D nanopillar array swab to the ANCA method, it is expected that pathogens (including strains and viruses, etc.) including antibiotic-resistant strains can be precisely diagnosed without the risk of secondary infection.

In addition, in the kit, the optimal amount of reagents used in a specific reaction can be easily determined by a person skilled in the art who has acquired the disclosure herein. Typically, the kit of the present disclosure is manufactured as a separate package or compartment comprising the aforementioned components.

In addition, the kit may further comprise instructions for use and other tools or equipment necessary for detection.

Meanwhile, the present disclosure provides a method for detecting a target nucleic acid comprising: reacting the composition for detecting a target nucleic acid described above with a sample. Specifically, the composition is a composition for detecting a target nucleic acid comprising: an Argonaute protein (Ago); a first guide nucleic acid (G1); a second guide nucleic acid (G2); a reporter sequence to which a quencher and a fluorophore are coupled; and a reporter complement, wherein the reporter complement consists of the same sequence as the reporter sequence to which the quencher and the fluorophore are not coupled, and wherein a phosphate is attached to the 5' end of the first guide nucleic acid, the second guide nucleic acid, or both.

In one embodiment of the present disclosure, optimal conditions for the ANCA reaction were established. Therefore, the reaction according to the present disclosure is preferably performed at 65 to 75°C, and most preferably at 75°C.

The sample according to the present disclosure refers to a biological sample and any gene (nucleic acid) derived therefrom. The term "biological sample" refers to any sample containing any DNA, RNA, and/or target DNA or RNA. The biological sample may be any tissue or body fluid obtained from a subject. The biological sample includes, but is not limited to, sputum, blood, serum, plasma, blood cells (e.g., leukocytes), tissues, biopsy samples, smear samples, washing samples, swab samples, cell-containing body fluids, free-floating nucleic acids, urine, peritoneal fluid and pleural fluid, cerebrospinal fluid, feces, lacrimal fluid, or cells derived therefrom, or pathogens (including strains and viruses, etc.) containing any nucleic acid of a subject. The biological sample may be obtained by a method that does not cause harm to the subject.

In particular, the composition and/or kit according to the present disclosure has excellent detection efficacy, enabling detection (diagnosis) of pathogens (including strains and viruses, etc.) present in objects as well as human samples even at very low concentrations.

Accordingly, the detection method of the present disclosure may further comprise: visually confirming a change in fluorescence development of a reactant; or measuring a change in fluorescence development of a reactant. The measurement of such a change in fluorescence development may be a conventional fluorescence measurement method in which the measurement wavelength of a fluorescence device is fixed. Specifically, it can be confirmed by fixing the measurement wavelength of the fluorescence equipment. For example, in the case of FAM fluorescence, a method of measuring the fluorescence intensity of a reactant at a wavelength of Ex: 495 nm / Em: 520 nm and observing the fluorescence change at 5 to 10 minute intervals for 1 to 2 hours may be used.

The detection method (ANCA method) according to the present disclosure has several notable advantages, particularly in the detection of pathogen (including strains and viruses, etc.) infection. First, unlike existing methods requiring separate nucleic acid amplification and extraction processes, the ANCA method simplifies the diagnostic process by detecting target nucleic acid (DNA) in a single step without an extraction step. Second, the reaction is performed isothermally. This feature reduces the need for complex and expensive thermal cycling equipment, making it easier and cheaper to use in a variety of settings. Third, it exhibits high sensitivity and specificity when detecting a target nucleic acid. This leads to the effect of improving detection accuracy and reducing the possibility of false positive or negative results. Fourth, since an Argonaute protein is used, a PAM sequence is not required for target recognition. This expands the range of detectable nucleic acid targets and increases utilization in diagnostic applications. Fifth, due to high stability, pathogens (including strains and viruses, etc.) can be directly detected even in harsh environments. Sixth, it can be easily integrated into portable diagnostic devices, enabling rapid on-site diagnosis of pathogen (including strains and viruses, etc.) infection. This is particularly useful in resource-limited settings or situations requiring immediate diagnosis.

### [Advantageous Effects]

The system according to the present disclosure can detect a target pathogen added to a human sample with 100% clinical sensitivity and specificity without nucleic acid amplification and separate extraction steps, and when used with a 3D nanopillar array structure, a target pathogen collected from the surface of an object can be directly identified. In addition, since the trigger sequence cleaved by the Argonaute protein is designed to be used as a guide, manufacturing is simple compared to existing nucleic acid detection circuits because a single protein is used; furthermore, unless the Argonaute protein, reporter, and reporter complement according to the system of the present disclosure are depleted, there is an advantage of cost reduction in that a target pathogen can be detected with high sensitivity even with a trace amount of target nucleic acid sequence. In addition, the system according to the present disclosure proceeds in a single step and has the advantage of being easily applicable to the detection of various target nucleic acid sequences by appropriately changing the nucleic acid recognition site to match the target pathogen.

### [Description of Drawings]

FIG. 1 is a schematic diagram showing the Argonaute protein and artificial nucleic acid circuit (ANCA) method according to the present disclosure.
FIG. 2 shows the results of T₀-T values of the Argonaute protein and artificial nucleic acid circuit (ANCA) method according to the forms of the reporter (R) and the reporter complement (R*) (T₀: Threshold time of a negative sample, T: Threshold time of a positive sample). Here, the threshold time is the reaction time at which the fluorescence intensity reaches 10,000.

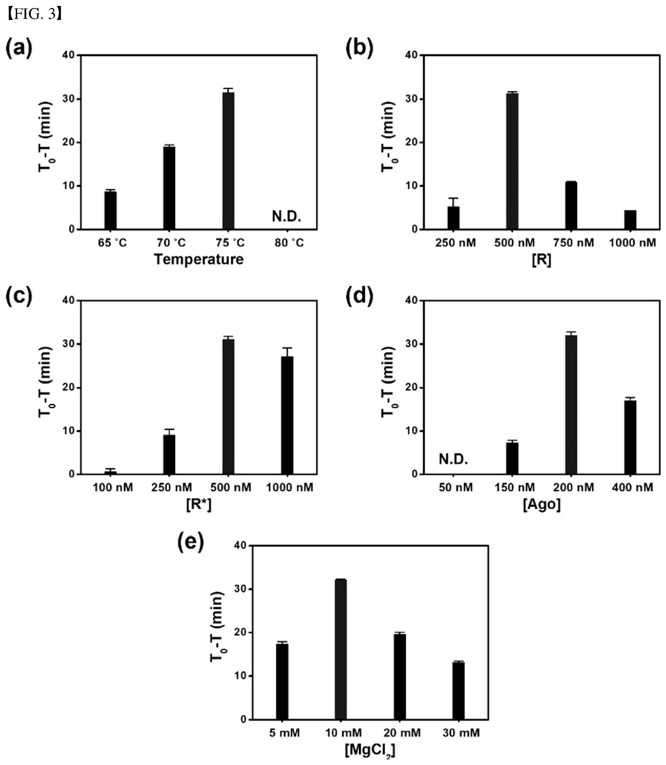

FIG. 3 shows the results of optimizing the reaction conditions of the Argonaute protein and artificial nucleic acid circuit (ANCA) method according to the present disclosure ((a): result of confirming optimal reaction temperature, (b): result of confirming optimal concentration of reporter (R), (c): result of confirming optimal concentration of reporter complement (R*), (d): result of confirming optimal concentration of Argonaute protein, (e): result of confirming optimal concentration of MgCl₂).

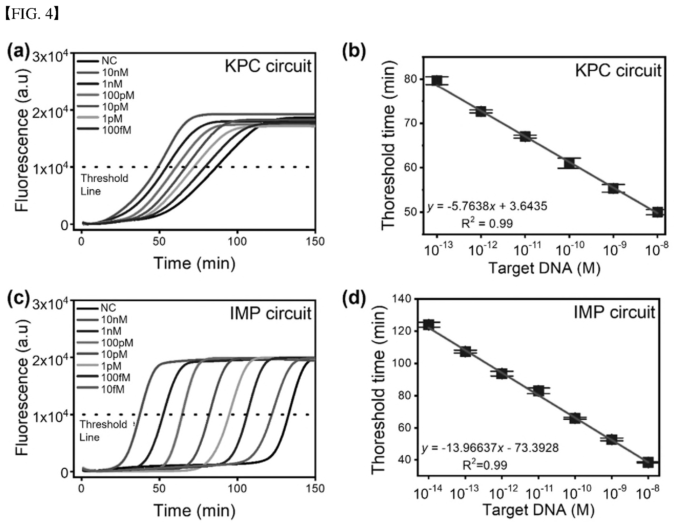

FIG. 4 shows the results of evaluating the sensitivity of the Argonaute protein and artificial nucleic acid circuit (ANCA) method according to the present disclosure to KPC (Klebsiella pneumoniae carbapenemase) and IMP (Klebsiella pneumoniae Imipenemase) ((a): result of confirming fluorescence intensity change over time for various concentrations of KPC, (b): result of confirming threshold time according to KPC concentration, (c): result of confirming fluorescence intensity change over time for various concentrations of IMP, (d): result of confirming threshold time according to IMP concentration). Here, the threshold time is the reaction time at which the fluorescence intensity reaches 10,000.

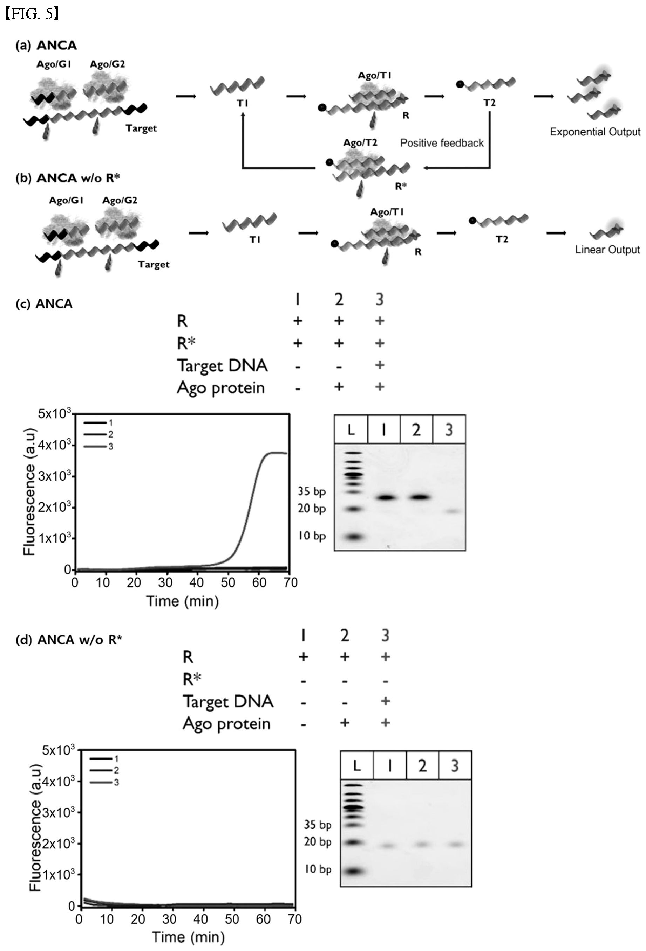

FIG. 5 shows the results of evaluating the Argonaute protein and artificial nucleic acid circuit (ANCA) method according to the present disclosure ((a): schematic diagram of ANCA according to the present disclosure, (b): schematic diagram of ANCA without reporter complement (R*), (c): target DNA detection result using ANCA (left panel: fluorescence intensity change according to reaction time, right panel: PAGE analysis result), (d): target DNA detection result using ANCA without reporter complement (R*) (left panel: fluorescence intensity change according to reaction time, right panel: PAGE analysis result)).

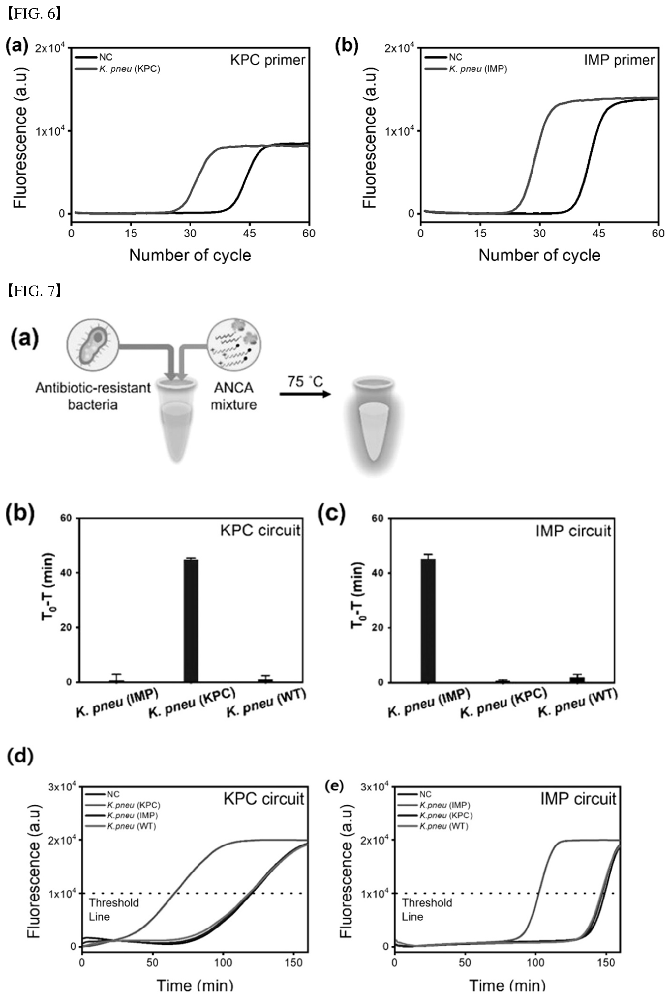

FIG. 6 shows RT-PCR results of detecting KPC and IMP positive (producing) strains using specific primers ((a): KPC detection, (b): IMP detection).
FIG. 7 shows the results of directly detecting antibiotic-resistant strains using the Argonaute protein and artificial nucleic acid circuit (ANCA) method according to the present disclosure ((a): ANCA reaction schematic diagram, (b): detection of KPC through ANCA method, (c): detection of IMP through ANCA method, (d): KPC detection through RT-PCR reaction, (e): IMP detection through RT-PCR reaction).

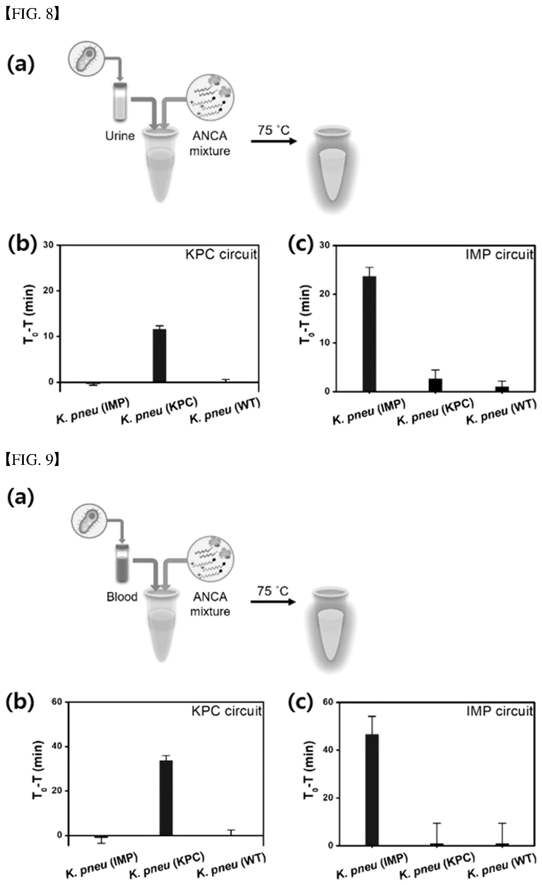

FIG. 8 shows the results of directly detecting antibiotic-resistant strains in urine samples using the Argonaute protein and artificial nucleic acid circuit (ANCA) method according to the present disclosure ((a): ANCA reaction schematic diagram, (b): detection of KPC through ANCA method, (c): detection of IMP through ANCA method).
FIG. 9 shows the results of directly detecting antibiotic-resistant strains in blood samples using the Argonaute protein and artificial nucleic acid circuit (ANCA) method according to the present disclosure ((a): ANCA reaction schematic diagram, (b): detection of KPC through ANCA method, (c): detection of IMP through ANCA method).

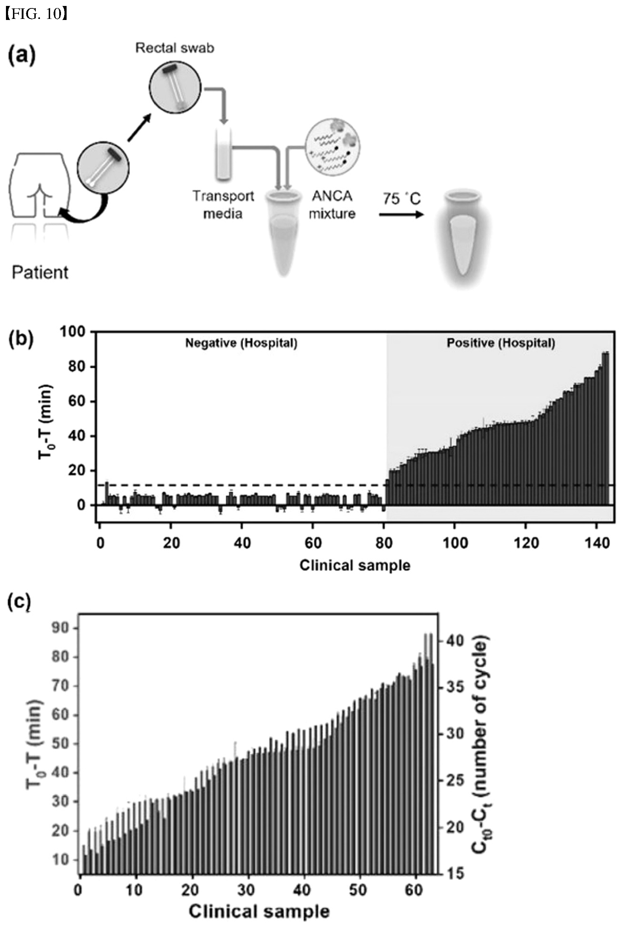

FIG. 10 shows the results of diagnosing antibiotic-resistant strains using the Argonaute protein and artificial nucleic acid circuit (ANCA) method according to the present disclosure ((a): schematic diagram of diagnosis of antibiotic-resistant strains in transport medium in which a rectal swab is immersed using the ANCA method, (b): result showing T₀-T values according to clinical samples (transport medium in which a rectal swab is immersed), (c): result showing T₀-T values (red) and Ct₀-Ct values (blue) for KPC-positive clinical samples). T₀ is the threshold time of the solution containing only the transport medium, and T is the threshold time of the clinical sample. Ct₀ was set to 50, and Ct represents the PCR threshold cycle value of the clinical sample.

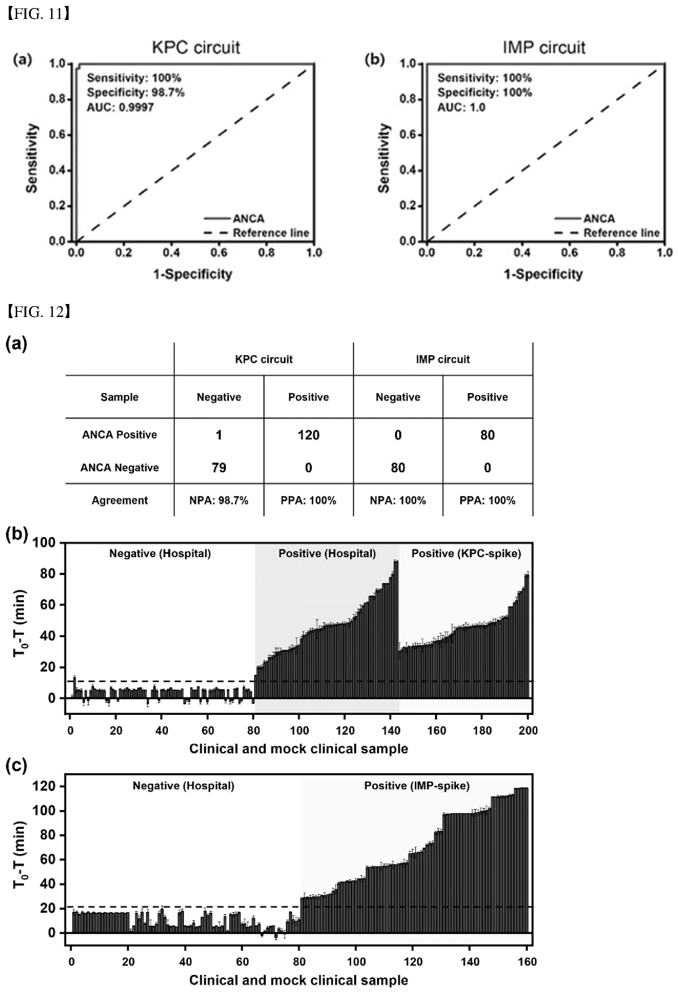

FIG. 11 shows ROC curves obtained from diagnostic results of clinical samples and samples added with antibiotic-resistant strains using the Argonaute protein and artificial nucleic acid circuit (ANCA) method according to the present disclosure ((a): evaluation of a total of 200 clinical samples (143) and KPC-added samples (57 transport media in which rectal swabs are immersed), (b): evaluation of a total of 160 clinical samples (80) and IMP-added samples (80 transport media in which rectal swabs are immersed)).
FIG. 12 shows the results of diagnosing clinical samples and samples added with antibiotic-resistant strains using the Argonaute protein and artificial nucleic acid circuit (ANCA) method according to the present disclosure ((a): result of calculating Negative Percent Agreement (NPA) and Positive Percent Agreement (PPA), (b): result showing T₀-T values for clinical samples and KPC-added samples (medium in which rectal swabs are immersed), (c): result showing T0-T values for clinical samples and IMP-added samples (medium in which rectal swabs are immersed)). T₀ is the threshold time for the transport medium to which nothing is added, and T is the threshold time for clinical samples and mock clinical samples.

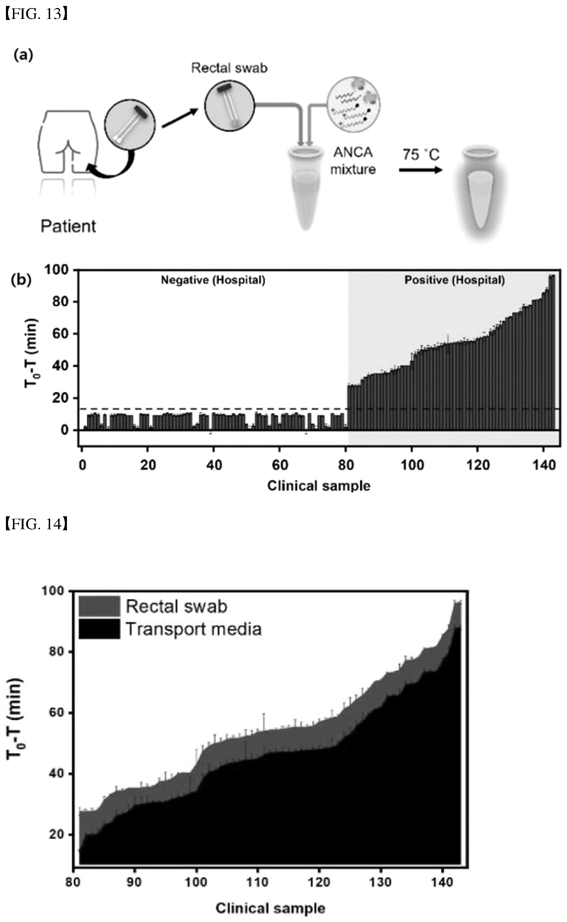

FIG. 13 shows the results of diagnosing infection of antibiotic-resistant strains directly from clinical samples (rectal swabs) using the Argonaute protein and artificial nucleic acid circuit (ANCA) method according to the present disclosure ((a): schematic diagram of diagnostic method, (b): result showing T₀-T values). T₀ is the threshold time of a clean rectal swab, and T is the threshold time of the clinical sample.
FIG. 14 is a result of comparing and plotting T₀-T values obtained from rectal specimens (red) and transport media (black) of KPC-positive clinical samples using the Argonaute protein and artificial nucleic acid circuit (ANCA) method according to the present disclosure. T₀ is the threshold time for the transport medium to which nothing is added, and T is the threshold time for the clinical sample.

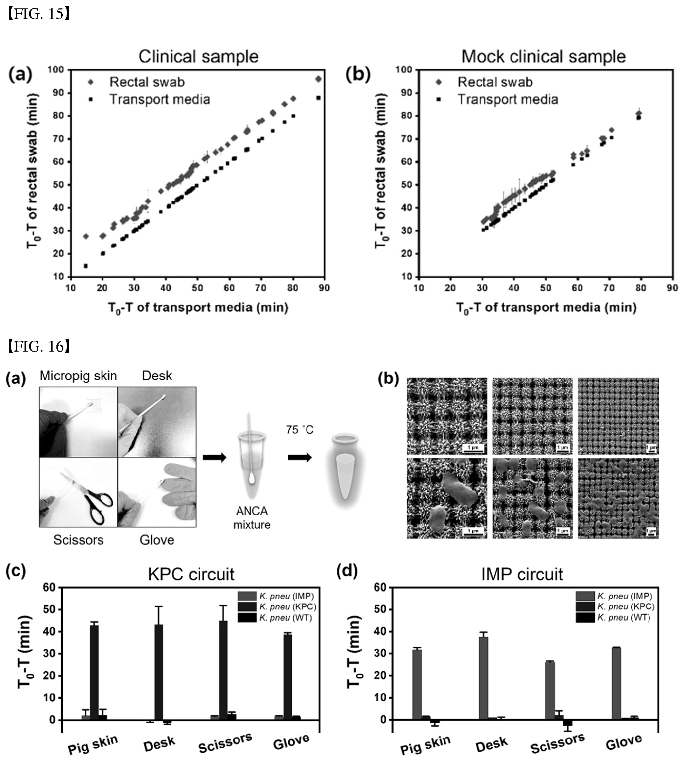

FIG. 15 shows scatter plots of T₀-T values obtained from rectal swabs (red) versus T₀-T values obtained from transport media (black) of KPC-positive clinical samples and mock clinical samples using the Argonaute protein and artificial nucleic acid circuit (ANCA) method according to the present disclosure ((a): clinical sample, (b): mock clinical sample).
FIG. 16 shows the results of detecting antibiotic-resistant bacteria collection using a 3D nanopillar swab and the Argonaute protein and artificial nucleic acid circuit (ANCA) method according to the present disclosure ((a): photograph and reaction schematic diagram of capturing antibiotic-resistant strains from micropig skin, desk, scissors, and gloves using a 3D nanopillar array swab, (b): Scanning Electron Microscope (SEM) images before (top) and after (bottom) collection of antibiotic-resistant strains using a nanopillar array swab, (c): result showing T₀-T values for KPC on contaminated surfaces, (d): result showing T₀-T values for IMP on contaminated surfaces). T₀ is the threshold time for the negative control sample, and T is the threshold time for the test sample.

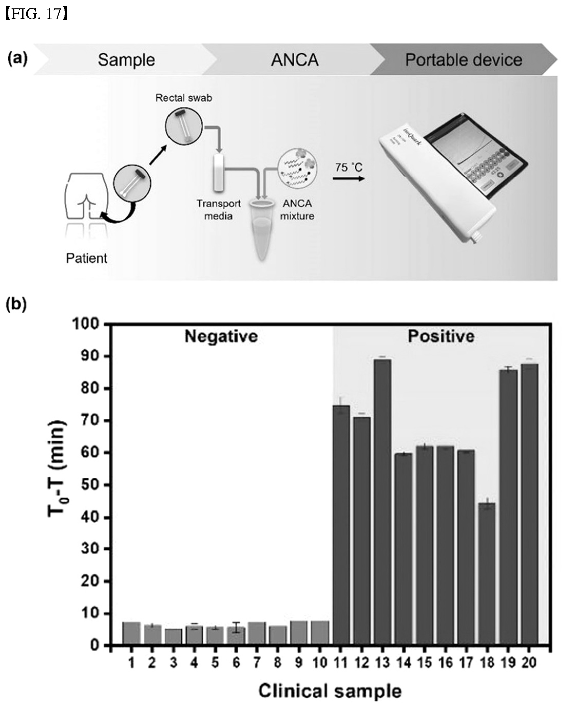

FIG. 17 shows the results of diagnosing infection of antibiotic-resistant strains for clinical specimens (transport medium in which a rectal swab is immersed) by applying the Argonaute protein and artificial nucleic acid circuit (ANCA) method according to the present disclosure to a portable device ((a): schematic diagram, (b): result of diagnosing antibiotic-resistant strain infection).

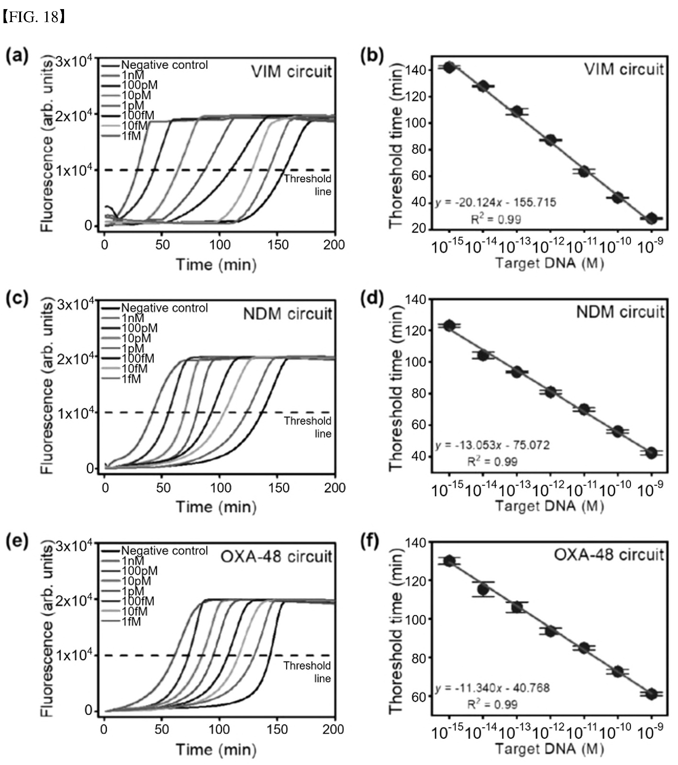

FIG. 18 shows the results of evaluating the sensitivity of the Argonaute protein and artificial nucleic acid circuit (ANCA) method according to the present disclosure to Verona integron-encoded metallo-beta-lactamase (VIM), New Delhi metallo-beta-lactamase (NDM), and oxacillinase-48 (OXA-48) ((a): result of confirming fluorescence intensity change over time for various concentrations of VIM, (b): result of confirming threshold time according to VIM concentration, (c): result of confirming fluorescence intensity change over time for various concentrations of NDM, (d): result of confirming threshold time according to NDM concentration, (e): result of confirming fluorescence intensity change over time for various concentrations of OXA-48, (f): result of confirming threshold time according to OXA-48 concentration). Here, the threshold time is the reaction time at which the fluorescence intensity reaches 10,000.

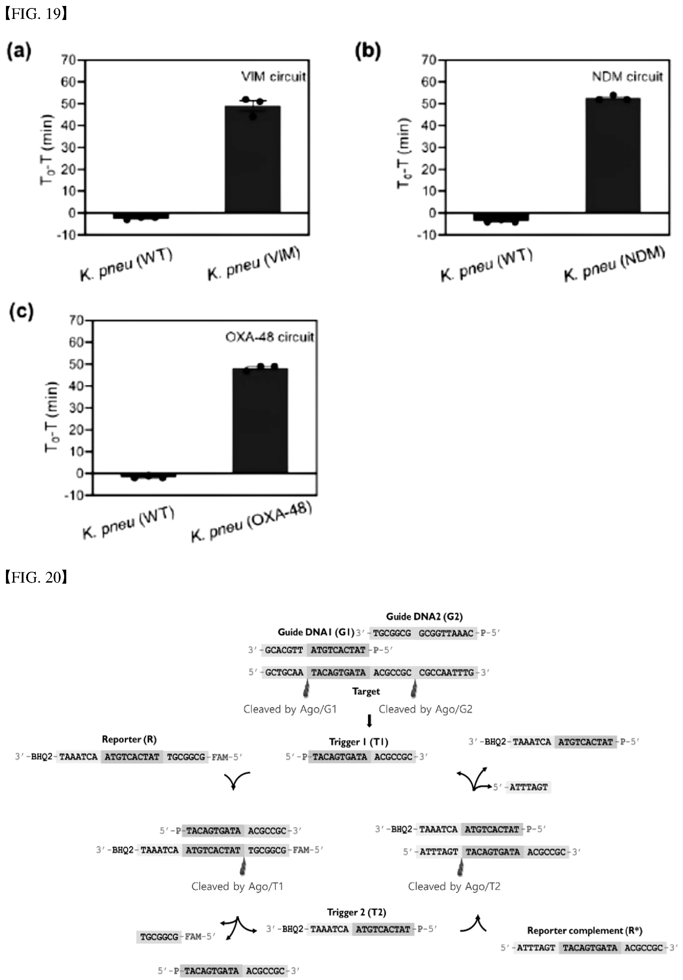

FIG. 19 confirms that each circuit can well distinguish between WT and CPKP as a result of attempting to detect carbapenemase-producing Klebsiella pneumoniae (CPKP) using VIM, NDM, and OXA-48 circuits.

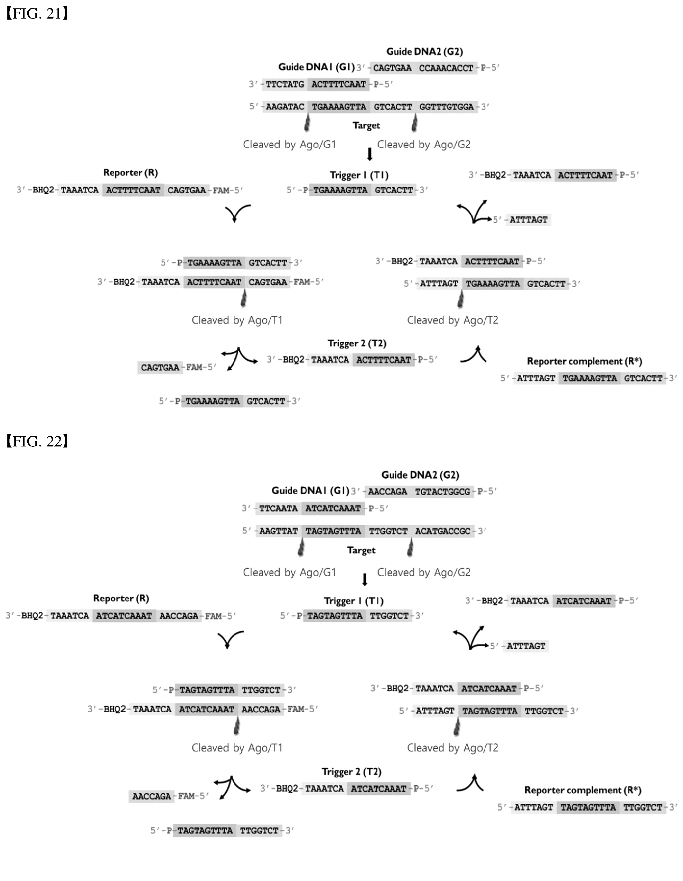

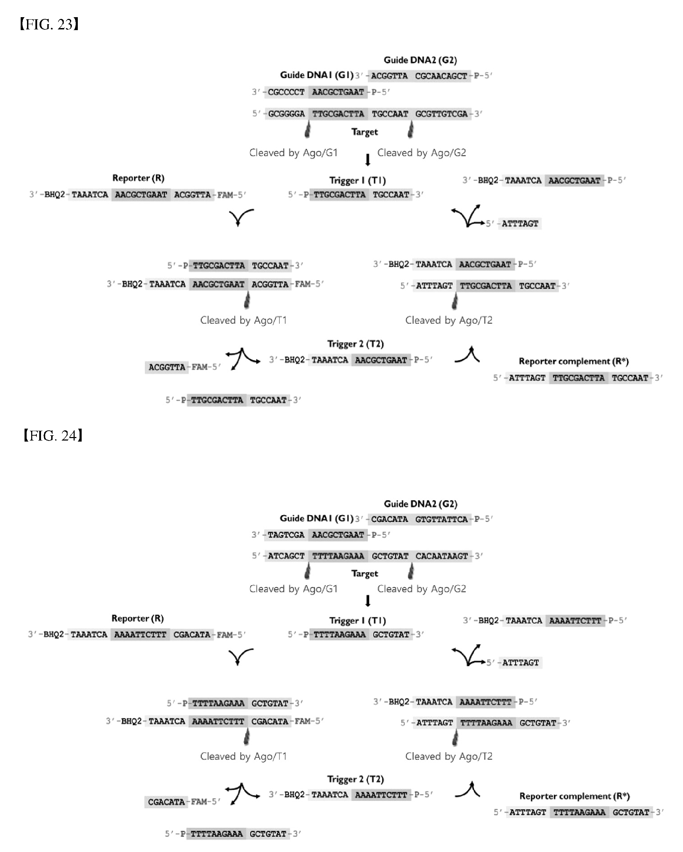

FIGS. 20 to 24 show schematic diagrams of individual systems. FIG. 20 relates to a sequence-based schematic diagram for the KPC circuit, FIG. 21 relates to a sequence-based schematic diagram for the IMP circuit, FIG. 22 relates to a sequence-based schematic diagram for the VIM circuit, FIG. 23 relates to a sequence-based schematic diagram for the NDM circuit, and FIG. 24 relates to a sequence-based schematic diagram for the OXA-48 circuit.

### [Description of Embodiments]

Hereinafter, the present disclosure will be described in more detail through examples. However, these examples are for illustrative purposes of the present disclosure, and the scope of the present disclosure is not limited to these examples.

### Experimental Materials and Methods

### 1 Experimental Materials

All oligonucleotides used in the present disclosure were purchased from Integrated DNA Technology (Coralville, IA, USA). TtAgo (Thermus thermophilus argonaute) was purchased from NEB (Beverly, MA, USA). Gel-Red Nucleic Acid Stain (41003) was purchased from Biotium (Fremont, CA, USA), and 6X DNA loading buffer and SYBR Green I Nucleic Acid Gel Stain were purchased from Takara Korea Biomedical Inc. (Seoul, Korea). TOPsimple preMIX (aliquot)-nTaq kit was purchased from Enzynomics (Daejeon, Korea), and human urine and blood samples were purchased from Innovative Research (Novi, MI, USA). Finally, the transport medium was purchased from Asanpharm (Seoul, Korea).

### 2 Evaluation of Argonaute Protein and Artificial Nucleic Acid Circuit (ANCA) Method

Target DNA (2 µl) for KPC (Klebsiella pneumoniae carbapenemase) was mixed with a reaction solution (18 µl). The reaction solution was configured to contain a reaction buffer containing Tris-HCl (20 mM), (NH₄)₂SO₄ (10 mM), KCl (10 mM), MgCl₂ (10 mM), and Triton X-100 (0.1%), and an Argonaute protein (TtAgo, 200 nM), a first guide nucleic acid (G1, 25 nM), a second guide nucleic acid (G2, 25 nM), a reporter sequence (R, 500 nM), and a reporter complement (R*, 500 nM). Then, this mixture was reacted at 75°C, and fluorescence signals were measured at 1-minute intervals using a CFX Opus 96 RT-PCR system (Bio-Rad, Hercules, CA, USA). The ANCA reaction without the reporter complement (R*) was performed under the same reaction conditions except for the reporter complement (R*).

In addition, for PAGE analysis, 1 nM of target DNA was combined with the ANCA reaction solution and reacted at 75°C for 70 minutes. Then, the reaction product was combined with 6X DNA loading buffer and loaded onto a 15% polyacrylamide gel. After electrophoresis, the gel was stained with Gel-Red Nucleic Acid Stain, and the visualized image was extracted using a Geldoc Go Imaging system (Bio-Rad).

### 3 Direct Detection of Antibiotic-Resistant Bacteria Using Argonaute Protein and Artificial Nucleic

### Acid Circuit (ANCA) Method

All bacterial strain clinical isolates were provided by Gyeongsang National University College of Medicine. Strains were cultured on LB (Luria-Bertani) agar medium at 37°C for 16 hours, and then harvested and resuspended in sterile PBS. The optical density (OD₆₀₀) of the obtained suspension was measured using a Nanophotometer P330 (Implen, Germany), and the number of strains was estimated from this value.

In order to confirm the presence of CPE-causing genes in the strains, gene-specific primers for each KPC (Klebsiella pneumoniae carbapenemase) and IMP (Klebsiella pneumoniae Imipenemase) gene were designed using Primer 3 tool (http://primer3.wi.mit.edu) and NCBI Reference sequence Database (https://www.ncbi.nlm.nih.gov/nuccore) GCF_000240185.1 (WT), NG_049244.1 (KPC), MH909334 (IMP), AAY33963.1 (VIM), AFN84620.1 (NDM), JN626286.1 (OXA-48)).

Then, qRT-PCR analysis was performed using these primers. Specifically, the antibiotic-resistant strains were combined with a PCR reaction solution containing gene-specific primer sets (0.5 µM each), 1X TOPsimple preMIX (aliquot)-nTaq, and 1X SYBR Green I dye to perform qRT-PCR. The amplification reaction proceeded through the following steps: A series of processes of denaturation at 95°C for 30 seconds, annealing at 55°C for 1 minute, and extension at 72°C for 1 minute was repeated a total of 50 times. Fluorescence signals of PCR were measured at each extension step using a CFX Opus 96 RT-PCR system.

For direct detection of antibiotic-resistant strains, 10⁵ cells/ml of strains were combined with ANCA reaction components and reacted according to the above-mentioned procedure. Each strain sample was individually mixed with KPC, IMP, VIM, NDM, and OXA-48 circuit mixtures.

To confirm the applicability of the ANCA method for strain detection in clinical samples, each strain was added to human urine and blood samples (10⁵ cells/mL). The urine and blood samples to which the strains were added were mixed with KPC (Klebsiella pneumoniae carbapenemase) and IMP (Klebsiella pneumoniae Imipenemase) circuit mixtures, which were treated according to the same procedure described above.

### 4 Diagnosis of Antibiotic-Resistant Bacteria Using Argonaute Protein and Artificial Nucleic Acid Circuit (ANCA) Method

143 clinical samples were obtained from Gyeongsang National University College of Medicine (63 KPC positive, 80 negative). Rectal specimens were collected from patients using swabs, placed in transport media (5 ml), and stored at -80°C until further use. This study was strictly reviewed and approved by the Institutional Review Board of Gyeongsang National University Changwon Hospital (IRB approval number 2022-10-012).

For the diagnosis of antibiotic-resistant strains, the transport medium (2 µl) in which the rectal swab was immersed was mixed with ANCA reaction components, and the reaction proceeded according to the aforementioned procedure. For comparison of ANCA method and qRT-PCR results, the transport medium in which the rectal swab was immersed was mixed with a PCR reaction mixture containing KPC (Klebsiella pneumoniae carbapenemase) gene-specific primer sets (0.5 µM each), 1X TOPsimple preMIX (aliquot)-nTaq, and 1X SYBR Green I dye. Fluorescence signals at each extension step were measured using a CFX Opus Real-Time System (Bio-Rad), and related software (CFX Maestro) was used to obtain Cₜ values.

To prepare mock clinical samples for KPC (Klebsiella pneumoniae carbapenemase), rectal swabs determined as negative at the hospital were randomly selected from those stored in transport media. Then, various concentrations of KPC (Klebsiella pneumoniae carbapenemase) were randomly sprayed onto these swabs, which were then immediately immersed in clean transport media. Finally, the prepared mock clinical sample (2 µl) was mixed with ANCA reaction components, and the reaction proceeded according to the aforementioned procedure.

To prepare mock clinical samples for IMP (Klebsiella pneumoniae Imipenemase), transport media (500 µl each) in which rectal swabs determined as negative at the hospital were immersed were randomly collected. Then, various concentrations of IMP (Klebsiella pneumoniae Imipenemase) were randomly mixed with the transport media. Finally, the prepared mock clinical sample (2 µl) was mixed with ANCA reaction components, and the reaction proceeded according to the aforementioned procedure.

For the diagnosis of antibiotic-resistant strains, rectal swabs collected from patients and mock rectal swabs of IMP (Klebsiella pneumoniae Imipenemase) were immersed in distilled water (500 µl). Then, the distilled water (2 µl) in which the rectal swab was immersed was mixed with ANCA reaction components, and the reaction proceeded according to the aforementioned procedure.

A portable isothermal nucleic acid amplification device was purchased from Revosketch (Daejeon, Korea). 20 transport media (2 µl each) in which rectal swabs were immersed were mixed with ANCA reaction components, and the reaction proceeded according to the aforementioned procedure.

### 5 Collection and Detection of Antibiotic-Resistant Bacteria Using Argonaute Protein and Artificial Nucleic Acid Circuit (ANCA) Method and 3D Nanopillar Swab

Each strain (10⁵ cells/mL) was sprayed onto Micropig Franz Cell Membrane (APURES, Seoul, Korea), a desk, scissors, and gloves. Then, strains present on the contaminated surfaces were collected by lightly rubbing using a 3D nanopillar array swab. SEM images were taken using a Nova 230 system (FEI, Hillsboro, OR, USA) at an accelerating voltage of 15 keV. The 3D nanopillar array on which the strains were collected was cut into a size of 0.25 cm X 0.25 cm capable of entering a PCR tube and input into the ANCA reaction solution. Then, the reaction proceeded according to the aforementioned protocol.

### Example 1. Design of Argonaute Protein and Artificial Nucleic Acid Circuit (ANCA) Method

The Argonaute protein and artificial nucleic acid circuit (ANCA) method was designed to construct a circulating circuit by fully utilizing two cleavage activities of the Argonaute protein, wherein 1) the cleavage site of the Argonaute protein is designed to be at the 10th and 11th positions from the 5' end of the guide DNA, and 2) since a phosphate group is attached to the 5' end of the strand cleaved by the Argonaute protein, the cleaved strand can be utilized as a guide DNA. The overall procedure of the ANCA method is shown in FIG. 1.

Specifically, in order to implement the ANCA method, four signal processors including a first guide nucleic acid (G1), a second guide nucleic acid (G2), a reporter (R), and a reporter complement (R*) were designed to operate cooperatively. The first guide nucleic acid (G1) and the second guide nucleic acid (G2) were designed according to the guide DNA design guidelines of the New England Biolabs Inc. (NEB) website, and the reporter (R) was designed to have both a quencher and a fluorophore. In addition, the sequences of all signal processors were delicately adjusted so that Trigger 1 (T1) and Trigger 2 (T2) generated during the reaction process could be used as guide DNAs for the Argonaute protein. The sequences of oligonucleotides used in this experiment are shown in Table 1 below. According to one embodiment, FAM was used as the fluorophore and BHQ2 was used as the quencher. In addition, details regarding each reaction are individually shown in FIGS. 20 to 24. FIG. 20 relates to a sequence-based schematic diagram for the KPC circuit, FIG. 21 relates to a sequence-based schematic diagram for the IMP circuit, FIG. 22 relates to a sequence-based schematic diagram for the VIM circuit, FIG. 23 relates to a sequence-based schematic diagram for the NDM circuit, and FIG. 24 relates to a sequence-based schematic diagram for the OXA-48 circuit.

**[Table 1]**

| **Name** | **Sequence (5'** -> **3')** | **Target** |
|---|---|---|
| KPC guide DNA 1 (G1) | P^{(a)}-TATCACTGTA TTGCACG (SEQ ID NO. 1) | KPC |
| KPC guide DNA 1 (G2) | P^{(a)}-CAAATTGGCGG CGGCGT (SEQ ID NO. 2) | |
| KPC Reporter (R) | GCGGCGT TATCACTGTA ACTAAAT (SEQ ID NO. 3) | |
| KPC Reporter (R)-fluorophore modified | FAM-GCGGCGT TATCACTGTA ACTAAAT-BHQ2 (SEQ ID NO. 4) | |
| KPC Reporter complement (R*) | ATTTAGT TACAGTGATA ACGCCGE (SEQ ID NO. 5) | |
| KPC Reporter complement (R*)- fluorophore modified | BHQ2-ATTTAGT TACAGTGATAACGCCGE-FAM (SEQ ID NO. 6) | |
| KPC forward primer | GGTTCTGTGGTCACCCATCT (SEQ ID NO. 7) | |
| KPC reverse primer | TCCAGACGGAACGTGGTATC (SEQ ID NO. 8) | |
| KPC synthetic target DNA | GGTGGCGGAGCTGTCCGCGGCCGCCGTGCAA TACAGTGATA ACGCCG CCGCCAATTTGTTGC (SEQ ID NO. 9) | |
| KPC synthetic target complementary DNA | GCAACAAATTGGCGGCGGCGTTATCACTGTATTGCACGGCGGC CGCGGACAGCTCCGCCACC (SEQ ID NO. 10) | |
| IMP guide DNA 1 (G1) | P^{(a)}-TAACTTTTCA GTATCTT (SEQ ID NO. 11) | IMP |
| IMP guide DNA 2 (G2) | P^{(a)}-TCCACAAACC AAGTGAC (SEQ ID NO. 12) | |
| IMP Reporter (R) | FAM-AAGTGAC TAACTTTTCA ACTAAAT-BHQ2 (SEQ ID NO. 13) | |
| IMP Reporter complement (R*) | ATTTAGT TGAAAAGTTA GTCACTT (SEQ ID NO. 14) | |
| IMP forward primer | CCTAAACATGGCTTGGTGGT (SEQ ID NO. 15) | |
| IMP reverse primer | GCATACGTGGGGATAGATCG (SEQ ID NO. 16) | |
| IMP synthetic target DNA | CTAATTGACACTCCATTTACGGCTAAAGATACTGAAAAGTTAGT CACTTGGTTTGTGGAGCGTG (SEQ ID NO. 17) | |
| IMP synthetic target complementary DNA | CACGCTCCACAAACCAAGTGACTAACTTTTCAGTATCTTTAGCC GTAAATGGAGTGTCAATTAG (SEQ ID NO. 18) | |
| VIM guide DNA 1 (G1) | P^{(a)}-TAAACTACTA ATAACTT (SEQ ID NO. 19) | VIM |
| VIM guide DNA 2 (G2) | P^{(a)}-GCGGTCATGT AGACCAA (SEQ ID NO. 20) | |
| VIM Reporter (R) | FAM-AGACCAA TAAACTACTA ACTAAAT-BHQ2 (SEQ ID NO. 21) | |
| VIM Reporter complement (R*) | ATTTAGT TAGTAGTTTA TTGGTCT (SEQ ID NO. 22) | |
| VIM trigger (T1) | TAGTAGTTTA TTGGTCT (SEQ ID NO. 23) | |
| VIM synthetic target DNA | ATGTTAAAAGTTATTAGTAGTTTATTGGTCTACATGACCGCGTCT GTCATGGCTGTCGCAAGTC (SEQ ID NO. 24) | |
| VIM synthetic target complementary DNA | GACTTGCGACAGCCATGACAGACGCGGTCATGTAGACCAATAA ACTACTAATAACTTTTAACAT (SEQ ID NO. 25) | |
| NDM guide DNA 1 (G1) | P^{(a)}-TAAGTCGCAA TCCCCGC (SEQ ID NO. 26) | NDM |
| NDM guide DNA 2 (G2) | P^{(a)}-TCGACAACGC ATTGGCA (SEQ ID NO. 27) | |
| NDM Reporter (R) | FAM-ATTGGCA TAAGTCGCAA ACTAAAT-BHQ2 (SEQ ID NO. 28) | |
| NDM Reporter complement (R*) | ATTTAGT TTGCGACTTA TGCCAAT (SEQ ID NO. 29) | |
| NDM trigger (T1) | TTGCGACTTA TGCCAAT (SEQ ID NO. 30) | |
| NDM synthetic target DNA | GGTATGGACGCGCGCTGCATGCGGCGGGATTGCGACTTATGCC AATGCGTTGTCGAACCAGCTTG (SEQ ID NO. 31) | |
| NDM synthetic target complementary DNA | CAAGCTGGTTCGACAACGCATTGGCATAAGTCGCAATCCCCGC CGCATGCAGCGCGTCCATACC (SEQ ID NO. 32) | |
| OXA-48 guide DNA 1 (G1) | P^{(a)}-TTTCTTAAAA AGCTGAT (SEQ ID NO. 33) | OXA-48 |
| OXA-48 guide DNA 2 (G2) | P^{(a)}-acttattgtg atacagc (SEQ ID NO. 34) | |
| OXA-48 Reporter (R) | FAM-ATACAGC TTTCTTAAAA ACTAAAT-BHQ2 (SEQ ID NO. 35) | |
| OXA-48 Reporter complement (R*) | ATTTAGT TTTTAAGAAA GCTGTAT (SEQ ID NO. 36) | |
| OXA-48 trigger (T1) | TTTTAAGAAA GCTGTAT (SEQ ID NO. 37) | |
| OXA-48 synthetic target DNA | ATTCGAATTTCGGCCACGGAGCAAATCAGCTTTTTAAGAAAGCT GTATCACAATAAGT TACACG (SEQ ID NO. 38) | |
| OXA-48 synthetic target complementary DNA | CGTGTAACTTATTGTGATACAGCTTTCTTAAAAAGCTGATTTGCT CCGTGGCCGAAATTCGAAT (SEQ ID NO. 39) | |
| (a) P indicates the modification of phosphate group at 5' end. | | |

### Example 2. Optimization and Sensitivity Evaluation of Argonaute Protein and Artificial Nucleic Acid Circuit (ANCA) Method

To determine the optimal conditions for the ANCA method, we first observed the change in fluorescence intensity according to the structures of the reporter (R) and the reporter complement (R*). As shown in FIG. 2, when the fluorophore and the quencher were attached only to the reporter (R), the ANCA method showed excellent target DNA detection performance. On the other hand, when the fluorophore and the quencher were attached to both the reporter (R) and the reporter complement (R*), the reaction efficiency significantly decreased, which is presumed to be due to steric hindrance interfering with the cleavage activity of the Ago/guide DNA complex.

Next, the reaction temperature and the concentrations of the reporter (R), reporter complement (R*), Argonaute protein, and Mg²⁺ were optimized. As shown in FIG. 3, conditions for obtaining optimal detection performance were confirmed to be a reaction temperature of 75°C, 500 nM of reporter (R) and reporter complement (R*) each, 200 nM of Argonaute protein, and 10 mM of MgCl₂. Therefore, these conditions were used for subsequent experiments.

Meanwhile, we intended to evaluate the sensitivity of the ANCA method for KPC (Klebsiella pneumoniae carbapenemase) and IMP (Klebsiella pneumoniae Imipenemase), which are target base sequences, respectively, under the optimized conditions as described above.

As shown in FIG. 4, a linear relationship with an R² value of 0.99 was observed in the range of 100 fM to 10 nM for KPC (Klebsiella pneumoniae carbapenemase), and a linear relationship with an R² value of 0.99 was observed in the range of 10 fM to 10 nM for IMP (Klebsiella pneumoniae Imipenemase).

Based on the above results, the limit of detection (LOD) of the ANCA method according to the present disclosure was calculated using the following formula, and the limit of detection (LOD) of the ANCA method according to the present disclosure was found to be 2.5 fM: (LOB = mean of negative samples + 1.645 × standard deviation of negative samples, LOD = LOB + 1.645 × standard deviation of low concentration samples).

The LOD was calculated to be 2.5 fM, which appeared to be much superior to previous results using Argonaute proteins without pre-amplification. In addition, since the probes used in the ANCA method according to the present disclosure successfully detected KPC and IMP sequences only by slightly modifying the sequences, these results can be seen as demonstrating the flexibility and broad target applicability of the ANCA method.

### Example 3. Evaluation of Argonaute Protein and Artificial Nucleic Acid Circuit (ANCA) Method

Using the optimal reaction conditions confirmed in Example 2, we intended to evaluate the ANCA method according to the present disclosure.

To this end, as shown in FIG. 5(a), the ANCA method was developed to achieve exponential fluorescence signal amplification through a positive feedback circuit. We first intended to evaluate how the results of the ANCA reaction vary depending on the components, using KPC (Klebsiella pneumoniae carbapenemase) as a target substance.

As shown in the red spectrum of FIG. 5(c), when all reaction components of ANCA including the target DNA were present, a strong fluorescence signal was observed during the ANCA reaction. On the other hand, as shown in the black and blue spectra of FIG. 5(c), in the absence of the target DNA and/or Ago protein, a negligible level of fluorescence signal was observed.

In order to more closely verify the validity of the ANCA method, polyacrylamide gel electrophoresis (PAGE) analysis was additionally performed. As shown in the right panel of FIG. 5(c), when the target DNA was absent or the Argonaute protein was absent, a band indicating an intact reporter (R)-reporter complement (R*) structure was confirmed before the ANCA reaction (Lanes 1 and 2 in FIG. 5(c)); however, after the intact ANCA reaction in which both the target DNA and ANCA reaction elements were present, it was confirmed that the band indicating the reporter (R)-reporter complement (R*) structure was completely degraded (Lane 3 in FIG. 5(c)).

Next, as shown in FIG. 5(b), a comparative experiment was performed to evaluate the amplification efficiency of the ANCA method. This will be described in detail as follows. In the ANCA reaction without the reporter complement (R*), the Ago/G1 and Ago/G2 complexes cleave the target DNA to form an Ago/Trigger 1 (T1) complex. Thereafter, the Ago/T1 complex cleaves the reporter (R) to generate a reaction product (Output) and Trigger 2 (T2).

However, as shown in the red spectrum of FIG. 5(d), since no additional reaction occurred due to the absence of the reporter complement (R*), the fluorescence signal did not increase any further during the 70-minute reaction even in the presence of the target nucleic acid sequence. In addition, as shown in the black and blue spectra of FIG. 5(d), the fluorescence signal did not increase without the target DNA and/or the Argonaute protein. These results were also confirmed in the PAGE results, as shown in the right panel of FIG. 5(d), where bands indicating the reporter (R) of an intact structure that was not cleaved were confirmed under all conditions.

Therefore, these results mean that the ANCA method is composed of a positive feedback circuit, providing high amplification efficiency and facilitating sensitive detection of target DNA.

### Example 4. Direct Detection of Antibiotic-Resistant Strains Using Argonaute Protein and Artificial Nucleic Acid Circuit (ANCA) Method

### 1 Direct Detection of Antibiotic-Resistant Strains

In this experiment, we attempted to detect antibiotic-resistant strains (CPE) without a separate DNA purification process using the ANCA method. To prevent cross-contamination, KPC-producing K. pneumoniae (hereinafter, KPC-positive (producing) strain) and IMP-producing K. pneumoniae (hereinafter, IMP-positive (producing) strain) were cultured separately and used for the experiment. As shown in FIG. 6, as a result of performing PCR using primers specific to KPC and IMP gene sequences, it was confirmed that the strains were well cultured.

Next, to verify the ANCA method according to the present disclosure, as shown in FIG. 7(a), antibiotic-resistant strains were directly added to a tube containing the ANCA reaction mixture and reacted at 75°C. As shown in FIG. 7(b), the largest T₀-T value was observed when KPC (Klebsiella pneumoniae carbapenemase) was present in the sample (blue bar), which is a result proving that the target antibiotic-resistant strain can be directly detected using the ANCA reaction for KPC. In addition, as shown in FIG. 7(c), it was confirmed that IMP (Klebsiella pneumoniae Imipenemase) could also be directly detected like KPC. This result can also be confirmed through FIGS. 7(d) and (e), in which real-time fluorescence was checked from RT-PCR using primers specific to KPC and IMP gene sequences.

The above results mean that using the ANCA method allows for the detection of antibiotic-resistant strains in a very simple manner without pre-concentration, lysis, and DNA purification steps. It is judged that this is because the ANCA method shows high activity at a high reaction temperature of 75°C.

### 2 Detection of Antibiotic-Resistant Strains from Clinical Samples

To confirm whether antibiotic-resistant strains can be detected even in clinical samples using the ANCA method, human urine and blood samples to which antibiotic-resistant strains were added were prepared.

As shown in FIG. 8(a) and FIG. 9(a), urine and blood samples to which strains were added were directly added to test tubes containing the ANCA reaction mixture and reacted.

As shown in FIGS. 8(b) and (c), KPC-producing strains (Klebsiella pneumoniae carbapenemase) and IMP-producing strains (Klebsiella pneumoniae Imipenemase) contained in urine (99%) were specifically detected.

In addition, as shown in FIGS. 9(b) and (c), KPC-producing strains (Klebsiella pneumoniae carbapenemase) and IMP-producing strains (Klebsiella pneumoniae Imipenemase) contained in blood (99%) were specifically detected.

These results suggest that the ANCA method can be used to detect antibiotic-resistant strains in harsh conditions. Considering that monitoring antibiotic-resistant strains in urine and blood samples can obtain valuable epidemiological data regarding the prevalence and distribution of antibiotic-resistant bacteria, these results have significant meaning in diagnostic and biological fields.

### Example 5. Diagnosis of Antibiotic-Resistant Strains Using Argonaute Protein and Artificial Nucleic Acid Circuit (ANCA) Method

### 1 Detection of Antibiotic-Resistant Strains Targeting Clinical Samples

Standard diagnostic procedures for antibiotic-resistant strains include several steps such as sample collection, strain culture, strain identification and isolation, antibiotic susceptibility testing, and carbapenem resistance determination. Since the entire series of processes takes several days, an efficient method capable of rapidly identifying antibiotic-resistant strains is needed. Generally, diagnosis of antibiotic-resistant strains is performed using rectal swabs collected from patients. In order to preserve the collected rectal swab samples intact, they are immersed in a transport medium immediately after collection. The transport medium is specially manufactured to maintain the integrity of strain samples during transport and storage, preserving the characteristics of the sample intact and preventing microbial overgrowth. We intended to diagnose antibiotic-resistant strains in clinical samples stored in transport media using the ANCA method.

To this end, a total of 143 rectal swab samples (63 KPC positive, 80 negative) were collected from each patient using swabs, and each swab was stored by immersing it in a transport medium. As shown in FIG. 10(a), the reaction was proceeded by mixing the transport medium in which the rectal swab was immersed with the KPC-specific ANCA reactant, and the change in fluorescence signal was observed. The cut-off T₀ - T value was calculated to be 10.11 using the following formula (cut-off T₀ - T value = average T₀ - T value of 80 negative samples + 5 × average standard deviation of 80 negative samples). The ANCA-based diagnostic test was conducted in a blind manner, and positive samples were marked in order from #81 to #143 according to the T₀ - T value after the experiment.

As shown in FIG. 10(b), the ANCA method was able to accurately distinguish 63 KPC positive samples and 79 negative samples based on the cut-off line.

In addition, as a result of comparative analysis of the diagnostic results of the ANCA method and PCR, as shown in FIG. 10(c), a strong correlation was observed between T₀ - T values and Ct₀ - Ct values for KPC-positive clinical samples. These results demonstrate that the ANCA method according to the present disclosure can be used for simple and accurate diagnosis of antibiotic-resistant strains.

### 2 Detection of Antibiotic-Resistant Strains Targeting Clinical Samples and Mock Clinical Samples

Meanwhile, in order to evaluate the clinical sensitivity and specificity of the ANCA method, 57 rectal swab specimens to which strains were added were utilized in addition to clinical samples. Considering that the clinical sensitivity and specificity of molecular diagnostics approved by the Korean Ministry of Food and Drug Safety (KFDA) are 95% respectively, at least 80 positive samples had to be tested using the ANCA method. Therefore, mock clinical samples were additionally prepared by adding KPC-producing strains (Klebsiella pneumoniae carbapenemase) to rectal specimen samples diagnosed as negative.

The amount of the added KPC-producing strains (Klebsiella pneumoniae carbapenemase) was determined randomly, and the experiment was conducted in a blind manner.

FIG. 11(a) shows the diagnostic results of a total of 200 clinical (143) and KPC-producing strain (Klebsiella pneumoniae carbapenemase) added samples (57), showing a sensitivity of 100% and a specificity of 98.7%. In particular, the Area Under the Curve (AUC) value appeared as 0.9997, which means that the ANCA method exhibits high accuracy even when compared to the PCR technique.

A chart for the T₀ - T values of the samples is shown in FIG. 12(b), and the mock clinical samples were marked in order from #144 to #200 based on the T₀ - T values after the experiment. These results are remarkable achievements considering that the ANCA method directly uses clinical samples without DNA purification and amplification steps.

In addition, a total of 160 clinical and mock clinical samples (80 IMP positive, 80 IMP negative samples) were used to verify the diagnostic ability of the IMP-specific ANCA reaction. Patients were monitored at the hospital for several months to collect IMP-positive rectal swab samples, but no infected patients were found. Therefore, IMP-positive samples were prepared by randomly adding IMP-producing strains (Klebsiella pneumoniae Imipenemase) to transport media in which rectal swabs diagnosed as negative were immersed.

As shown in FIG. 11(b), the clinical sensitivity and specificity for IMP-producing strains (Klebsiella pneumoniae Imipenemase) were both found to be 100% and 100%, and it was confirmed that the AUC was 1.0. A chart for the T₀ - T values of the samples is shown in FIG. 12(c).

### 3 Detection of Antibiotic-Resistant Strains Directly from Rectal Swabs

Judging from the previous results, the ANCA method was able to detect antibiotic-resistant strains even under difficult conditions due to its excellent stability. Accordingly, we further investigated whether KPC-producing strains (Klebsiella pneumoniae carbapenemase) could be detected by directly using swabs obtained from patients.

As shown in FIG. 13(a), after immersing the swab in a test tube containing the KPC-specific ANCA reactant, the reaction proceeded at 75°C.

As shown in FIG. 13(b), even when the rectal swab was directly used, the ANCA method succeeded in accurately identifying KPC-producing strains (Klebsiella pneumoniae carbapenemase). At this time, the diagnostic results obtained from rectal swabs (sensitivity = 100%, specificity = 100%) showed superior accuracy compared to the diagnostic results using transport media in which rectal swabs were immersed (sensitivity = 98.7%, specificity = 100%).

Furthermore, as shown in FIG. 14, a strong correlation was observed between the T₀-T values of KPC-positive rectal swabs and transport media. This suggests that collected antibiotic-resistant strains exist simultaneously in rectal swabs and transport media.

Interestingly, as shown in FIG. 15(a), the average T0-T value (54.89) of KPC-positive rectal swabs was higher than the average T₀-T value (47.02) of transport media. Through this, it can be inferred that more KPC-producing strains (Klebsiella pneumoniae carbapenemase) exist on rectal swabs than in transport media in which rectal swabs are immersed.

To quantitatively compare this difference, we calculated the difference in the amount of target DNA utilizing the calibration curve of the KPC circuit shown in FIG. 4(b). For convenience of calculation, it was assumed that the T₀-T value would show a similar trend to the y value of the calibration curve, and based on this, the following equation was derived: yₜ - yₛ = -5.7638(xt - xₛ), where yₜ and yₛ represent the T₀ - T values of the transport medium and the rectal swab, respectively, and xt and xₛ represent the logarithmic values of the KPC concentration of the transport medium and the rectal swab, respectively. Substituting yₜ = 47.02 and yₛ = 54.89 into this equation, the value of xt - xₛ = 1.365 can be derived. Here, since x represents a value derived from the logarithmic value of the target nucleic acid concentration, the actual concentration ratio between the two samples corresponds to 23.196. This means that KPC-producing strains can be detected more than 20 times more sensitively when rectal swabs are used directly.

As shown in FIG. 15(b), a similar trend was observed in the experimental results derived from mock clinical samples. In this comparative experiment, rectal swabs were first immersed in transport media before being used for the ANCA method. Considering that the volume of the transport medium (5 ml) is large enough to dilute the strain concentration, these results are consistent with logical expectations. However, the important point is that a significant number of strains remain on the rectal swab even after being immersed in the transport medium.

These experimental results suggest that the ANCA method has significant potential for directly detecting antibiotic-resistant strains from rectal swabs. Also, this means that the ANCA method can significantly contribute to obtaining accurate diagnostic results and establishing appropriate treatment strategies in medical environments.

### Example 6. Collection and Detection of Antibiotic-Resistant Strains Based on Argonaute Protein and Artificial Nucleic Acid Circuit (ANCA) Method Applying 3D Nanopillar Swab

Nosocomial infections can occur through various routes, such as direct or indirect contact with infected persons, respiratory droplets or airborne transmission, and transmission by other vectors. In particular, infection with antibiotic-resistant strains frequently occurs through contaminated skin and medical equipment. Therefore, regular monitoring of patients and contaminated surfaces can help in the early detection and control of potential antibiotic-resistant strain outbreaks. Since the ANCA method succeeded in detecting KPC- and IMP-producing strains in clinical specimens in the previous results, we set an additional goal of collecting and detecting antibiotic-resistant strains from contaminated surfaces using the ANCA method.

As shown in FIG. 16(a), a 3D nanopillar array swab was used in this experiment to efficiently collect strains present on the surface. For the experiment, KPC- and IMP-producing strains were intentionally sprayed onto micropig skin, a desk, gloves, and scissors. Then, the strains were collected by gently contacting and rubbing the swab on the contaminated surfaces. After that, the swab with collected strains was directly immersed in a test tube containing the ANCA reactant, and the ANCA reaction was performed. The complex 3D nanopillar array structure promotes the attachment of strains through nanotopography interactions, thereby irreversibly capturing the strains and preventing secondary infection.

FIG. 16(b) is a scanning electron microscope (SEM) image of the nanopillar array structure according to whether or not antibiotic-resistant strains were collected, showing that the antibiotic-resistant strains were successfully captured on the swab. As shown in FIG. 16(c), as a result of using the KPC-specific ANCA reactant, the fluorescence signal was rapidly amplified only when the KPC-producing strain (Klebsiella pneumoniae carbapenemase) was captured with the 3D nanopillar swab.

Similarly, as shown in FIG. 16(d), in the case of the IMP circuit, the signal increased rapidly only when the IMP-producing strain (Klebsiella pneumoniae Imipenemase) was captured. These results suggest that on-site collection and identification of antibiotic-resistant strains are possible using the ANCA method together with the 3D composite nanopillar array swab.

### Example 7. Diagnosis of Antibiotic-Resistant Strain Infection Based on Argonaute Protein and Artificial Nucleic Acid Circuit (ANCA) Method Applying Portable Isothermal Nucleic Acid Amplification Device

As shown in FIG. 17(a), the function of the ANCA method was further confirmed using a commercially available portable isothermal nucleic acid amplification device.

As shown in FIG. 17(b), as a result of testing a total of 20 clinical specimens (10 KPC positive, 10 negative), the ANCA method according to the present disclosure accurately diagnosed KPC positive specimens. These results demonstrate the versatility of the ANCA method in various experimental environments and the possibility of on-site detection of antibiotic-resistant strains.

### Example 8. Detection of New Delhi metallo-beta-lactamase (NDM), Verona integron-encoded metallo-beta-lactamase (VIM), and oxacillinase-48 (OXA-48) Using Argonaute Protein and Artificial Nucleic Acid Circuit (ANCA) Method

As described in Example 2, we intended to evaluate the sensitivity of the ANCA method for New Delhi metallo-beta-lactamase (NDM), Verona integron-encoded metallo-beta-lactamase (VIM), and oxacillinase-48 (OXA-48), which are target base sequences, respectively, under optimized conditions. A system was designed based on the sequences described in Example 1 above and evaluated.

As shown in FIG. 18, in all cases, a linear relationship (R² = 0.99) was observed in the range of 1 fM to 1 nM, and the LODs of VIM, NDM, and OXA-48 were calculated to be 529, 120, and 144 aM, respectively. This corresponds to a level much superior to previous results utilizing Ago proteins without pre-amplification. In addition, FIG. 18 shows that the present method has flexibility and broad target applicability, and shows the result of successfully detecting the target DNA sequence.

### Example 9. Direct Detection of Antibiotic-Resistant Bacteria

As in the method described in Example 4, an attempt was made to detect CPKP by the ANCA method without DNA purification.

The results are shown in FIG. 19.

As shown in FIGS. 19(a), (b), and (c), as a result of attempting to detect carbapenemase-producing Klebsiella pneumoniae (CPKP) using VIM, NDM, and OXA-48 circuits, it was confirmed that each circuit could well distinguish between WT and CPKP. That is, it was confirmed that CPKP could be detected in a simplified manner without the need for pre-concentration, lysis, and DNA purification steps using the ANCA method, and it was confirmed that there is a possibility of universal application. In particular, it was confirmed that the value of utilization is high in that detection is possible without complex, difficult, and time-consuming steps related to lysis and nucleic acid extraction if this method is utilized.

## Claims

1. A composition for detecting a target nucleic acid comprising: an Argonaute protein; a first guide nucleic acid (G1); a second guide nucleic acid (G2); a reporter sequence to which a quencher and a fluorophore are coupled; and a reporter complement, wherein the reporter complement consists of the same sequence as the reporter sequence to which the quencher and the fluorophore are not coupled, and wherein a phosphate is attached to a 5' end of the first guide nucleic acid, the second guide nucleic acid, or both.

2. The composition according to claim 1, wherein the first guide nucleic acid, the second guide nucleic acid, or both are designed so that Trigger 1 (T1) and Trigger 2 (T2), which are reaction products cleaved by the Argonaute protein, can act as separate guide nucleic acids.

3. The composition according to claim 1, wherein the first guide nucleic acid, the second guide nucleic acid, or both have one or more conditions selected from the following conditions:
1) consisting of 16 to 18 bases,
2) having a CG content between 10% and 30%,
3) a thymine is located at a first base of the nucleic acid, and
4) an adenine is not the 12th base of the nucleic acid.

4. The composition for detecting a target nucleic acid according to claim 1, wherein the quencher is any one selected from the group consisting of DABCYL, BHQ, ECLIPSE, and TAMRA.

5. The composition for detecting a target nucleic acid according to claim 1, wherein the fluorophore is any one selected from the group consisting of ALEX-350, FAM, VIC, TET, CAL Fluor Gold 540, JOE, HEX, CAL Fluor Orange 560, TAMRA, CAL Fluor Red 590, ROX, CAL Fluor Red 610, TEXAS RED, CAL Fluor Red 635, Quasar 670, CY3, CY5, CY5.5, and Quasar 705.

6. A kit for detecting a target nucleic acid comprising the composition according to any one of claims 1 to 5.

7. The kit according to claim 6, wherein the kit comprises 150 to 400 nM of the Argonaute protein; 250 to 1,000 nM of the reporter sequence; and 250 to 1,000 nM of the reporter complement.

8. The kit according to claim 6, wherein the kit further comprises a reaction buffer containing MgCl₂.

9. The kit according to claim 8, wherein the MgCl₂ has a concentration of 5 to 30 mM.

10. The kit according to claim 6, wherein the kit further comprises, as a collection tool for collecting a sample, at least one selected from the group consisting of an absorbent pad, a cotton swab, a pipette, a spoon, a swab, and a toothpick.

11. The kit according to claim 10, wherein the collection tool is a cotton swab.

12. A method for detecting a target nucleic acid comprising: reacting the composition for detecting a target nucleic acid according to any one of claims 1 to 5 with a sample.

13. The method according to claim 12, further comprising: visually confirming a change in fluorescence development of a reactant; or measuring a change in fluorescence development of a reactant.

14. The method according to claim 12, wherein the reaction is performed at 65 to 75°C.
